# EUROPEAN PATENT SPECIFICATION

(11) **EP 1 487 469 B1**
(45) Date of publication and mention of the grant of the patent: **08.10.2008**
(21) Application number: 03718098.1
(22) Date of filing: 28.03.2003
(51) Int. Cl.: A61K 36/53

(54) **CO-BEADLET OF DHA AND ROSEMARY**
CO-PERLCHEN VON DHA UND ROSMARIN
CO-GRANULE DE DHA ET DE ROMARIN

(30) Priority: 28.03.2002 US 368301 P
(43) Date of publication of application: 22.12.2004
(73) Proprietor: Alcon, Inc., 6331 Hünenberg (CH); Biodar Ltd., 81103 Yavne (IL)
(72) Inventor: LANG, John, C., Cedar Hill, TX 75104 (US); BLATT, Yoav, Rehovot (IL); KAZHDAN, Pavel, 81103 Yavne (IL); PINTO, Rika, 61000 Tel Aviv (IL); SAFRONCHIK, Oleg, Ashdod (IL)
(74) Representative: Moore, Barry
(86) International application number: PCT/US2003/009643
(87) International publication number: WO 2003/082313

(56) References cited:
- WO-A-94/01001
- WO-A-03/018186
- WO-A-03/082249
- DE-U1- 20 205 184
- US-A- 4 525 306
- DATABASE PROMT [Online] 'A bright outlook for European Sun Care', XP002966155 Database accession no. 2001:347645 & HOUSEHOLD & PERSONAL PRODUCTS INDUSTRY vol. 38, no. 4, April 2001, page 42

## Description

### BACKGROUND OF THE INVENTION

### 1. Field of the Invention

The present invention relates to the field of nutraceuticals and to the use of dietary supplements to maintain or improve ocular health.

### 2. Description of the Related Art

Dietary supplements are taken for a variety of reasons including the improvement of vision or prophylaxis of vision loss. Examples of a set of dietary supplements useful in promoting healthy eyes are the ICaps® Dietary Supplements (Alcon Laboratories, Inc., Fort Worth, TX). Dietary supplements are generally in the form of powders, tablets, capsules or gel-caps and comprise a variety of vitamins, minerals, and herbal, or other organic or botanical constituents. Some dietary supplements are formulated with beadlets, which may be incorporated in tablets or filled into capsules or gel caps.

Beadlets containing dietary substances are generally small spheroids of less than about a millimeter in diameter. There are a variety of functions and purposes of beadlets. For example, beadlets may provide for the separate containment of an ingredient or ingredients within the dietary supplement to improve the stability of the entrapped ingredients.

Various beadlet compositions are known and can be obtained from a number offood ingredient or pharmaceutical manufacturers including H. Reisman Corp. (Orange, NJ), BASF (Mount Olive, NJ) and Hoffmann-LaRoche (Nutley, NJ). Particular beadlet compositions have been the subject of several patents including U.S. Patent Nos. 4,254,100 (Keller et al.) and 3,998,753 (Antoshkiw et al.). Methods of beadlet manufacture have been disclosed in U.S. Patent Nos. 4,670,247 (Scialpi) and 3,998,753.

Current beadlet compositions used in dietary supplements generally are restricted to the use of inert ingredients and excipients complementary to a single nutritional compound, or "active ingredient," contained within the beadlet. In other instances, when molecules of the same class are refined from a particular source, for example a major component with a minor related constituent, and both compounds produce parallel effects, such molecules may not necessarily be isolated but mixed together in a beadlet. These may be considered pseudo-single-component beadlets. Current examples in the market place include Lutrinol® and FloraGLO® beadlets, which are a combination of lutein and zeaxanthin as formulated in Retoxil® Dietary Supplements. Examples of ingredients benefiting from beadlet confinement have included natural vitamins such as Vitamins A, D, E, and K; xanthophylls such as lutein, zeaxanthin, canthaxanthin, and astaxanthin; and carotenes, such as beta-carotene, lycopene, and retinol.

Alcon, Inc. (formerly known as Alcon Universal Ltd.) owns pending U.S. patent No. 6,582,721 and related foreign applications directed to beadlets containing more than one active ingredient. More specifically, those applications are directed to a beadlet containing one or more xanthophylls, one or more carotenes/retinoids, one or more antioxidants and one or more solidifying agents. The beadlets of US. patent No. 6, 582,721 are aimed at treating or preventing mammalian diseases or disorders. U.S. patent No. 6,582,721 does not discuss the use of docosahexaenoic acid (DHA) or rosemary alone or in any combination.

DHA is a long chain fatty acid known as an ω-3 essential fatty acid. It is found at relatively high concentrations in oily cold-water fish, such as salmon, herring or sardine, and may also be obtained from algae by fermentation. Omega-3 fatty acids balance the more common ω-6 fatty acids found in corn and soy oils as well as the fats in meats and dairy products. It is know that increasing intake of ω-3 fatty acids while decreasing intake of ω-6 fatty acids improves anti-inflammatory actions in the body and generally supports the immune system. DHA concentrates in the brain and retina and has been shown to be important in cognitive development and visual acuity, especially for premature babies (Birch, *et al*. 2000).

Unfortunately, DHA is also associated with an unpleasant "fishy," or "marine" odor. Therefore, although DHA is known to offer health benefits when ingested, it is not found in many nutritional products. The unpleasant odor discourages many consumers from using it. Thus, a product providing the health benefits of DHA without the associated odor is needed.

It is believed that the unpleasant odor associated with DHA results from the oxidative byproducts of the fatty acid. Beadlet technology has been used for protecting compounds from oxidation, e.g., lutein. This has been provided using a variety of known antioxidants, primarily derivatives of common water- and oil-soluble antioxidants known to be safe for extended human consumption, specifically tocopherols (Vitamin E-related compounds) and ascorbates (Vitamin C derivatives) such as ascorbyl palmitate. However, it has been found that, over time, bottled lutein-containing products behave in specific ways: (1) all of the oxygen in the head space eventually reacts with antioxidants; (2) any oxygen which diffuses through the plastic bottle also will react with the confined antioxidants; (3) the oxygen reacts preferentially with the most oxidizable antioxidants. Thus, while beadlet technology provides some protection from oxidation, it has not, by itself, been the ideal solution to the odor problem associated with DHA, or with the stability problem associated with other ingredients. Moreover, it has been shown that compression of beadlets containing DHA into tablets, the preferred form of administration of such dietary supplements, enhances the rate of generation of the unpleasant marine odor. An instability after compression has also been observed following compression of beadlets containing lutein.

Despite the disadvantages associated with products containing DHA, *i.e*., *unpleasant odor,* it would still be desirable to provide a nutritional product containing DHA for the improvement of ocular health. The provision in the same beadlet of other components that would provide additional ocular health benefits would also be desirable. Thus, what is needed is a beadlet formulation that is both odorless and stable, which does not deteriorate over the shelf life of the product, which improves ocular health, and which can withstand the compressive forces of tableting.

Examples of prior art compositions including DHA and antioxidants include those described in DE-U-20205184, US4525306 and WO9401001.

### SUMMARY OF THE INVENTION

The present invention overcomes these and other drawbacks of the prior art by providing improved beadlet formulations according to claim 1. In particular, the improved beadlets comprise DHA (docosahexaenoic acid); rosemary and/or its components; and excipients. The beadlets are particularly useful for incorporation in dietary supplements customized for improving and maintaining ocular nutrition.

The present invention is also directed to improved dietary supplements comprising the improved beadlets, especially when given in compressed tablets such as in claim 2. Preferred dietary supplements have been formulated as an aid to ocular health.

The application of the beadlet technology of the present invention to dietary supplements provides, and facilitates development of, enhanced nutritional supplementation. Such technology may aid in increasing availability of the dietary substances and also provide ease in modifying compositions containing DHA and rosemary within the supplement. Such improvements are believed to be particularly useful in the enhancement of ocular nutrition and improved ocular health.

### DETAILED DESCRIPTION PREFERRED EMBODIMENTS

The present invention is directed to improved beadlet formulations, improved dietary supplement formulations comprising the improved beadlets. As used herein, "dietary supplement(s)" or the shortened form, "supplement(s)," refer to any finished, dietary supplement dosage form containing dietary substances and suitable for ingestion by a host, e.g., human or other mammal. Specifically, the improved beadlet formulations of the present invention may enhance ocular nutrition and improve ocular health by providing a stable form of rosemary and DHA within a single beadlet, and thereby offering the likelihood of improved consumption compliance since the dosage form will be essentially odorless, having eliminated the marine odor generally found offensive.

Rosemary has been used for centuries to treat central nervous system disorders. More recently, rosemary has been used to enhance the senses and boost memory. It has also been shown to have properties beneficial to ocular health. Additionally, rosemary contains antioxidants, including carnosic acid, rosmarinic acid, and carnosol.

Rosemary utilized in this invention consists primarily of the lipid fraction from the leaves of *Rosmarinus officinalis* extracted utilizing solvents such as ethanol/water, propanol/water, or other multicomponent alcohol/water extracting agents or mixtures of nonpolar/polar organic solvents such as hexane/ethylacetate in which, after solvent evaporation the residue contains carnosic acid in concentrations greater than 0.5% and preferably greater than 3%.

Docosahexaenoic acid (DHA) is an ω-3 essential fatty acid derived from fish oil or from algae by fermentation. Both minor components of DHA and the by-products of its oxidation have been observed to possess an objectionable aroma, sometimes described as a "marine odor." Producers have attempted to eliminate this drawback of the use of DHA by devising a purer product or by creating carriers capable of protecting the product from oxidation. For example, recently both the algal fermentation process and the isolation of DHA from the broth have both been improved resulting in a DHA product containing algal-derived DHA that has significantly diminished marine odor relative to that obtained from fish oil. The invention described is intended for use with either DHA as the free acid, DHA esters, and even the DHA biomass. It also is to include sources of DHA, which may or may not include significant fractions of the ω-6 fatty acid EPA (eicosapentaenoic acid), in the corresponding forms.

To improve stability of a substantially odor-free DHA product and to maintain the more odor-free status, the DHA was encapsulated in a beadlet using known gelatin beadlet technology. Also, comparison of the algal-derived DHA-containing beadlets with fish oil-derived DHA-containing beadlets revealed a significant improvement in the odor. However, when the DHA-containing beadlets were compressed into tablets, the marine odor was again prevalent, regardless of the source of the DHA.

The present inventors have found that adding rosemary and/or its components to the DHA-containing beadlets, such that both "active" ingredients are present in a single beadlet, eliminates the odor generation while stabilizing the composition, thus increasing its shelf-life. Rosemary antioxidants are found to be oxidized preferentially to DHA. The combination of DHA and rosemary improves ocular health by providing at least two active ingredients within a single beadlet, both ingredients contributing to the enhancement or improvement of ocular health. Moreover, the rosemary present within the beadlet may also provide some protection to the DHA in the beadlet, allowing its health-improving properties to be maximized while diminishing the potential for an unpleasant odor. The rosemary may in part also protect other ingredients and other antioxidants that may be present in the entire tablet dosage form.

While it is contemplated that virtually any beadlet technology, such as that described in U.S. Patent Nos. 4,670,247 and 3,998,753 will be useful in the practice of the present invention, it is believed that the beadlet technology most useful for the practice of the present invention is the non-gelatin, alginate beadlet technology of Bio Dar, a subsidiary of LycoRed. The non-gelatin alginate beadlet technology useful for the practice of the present invention is the subject of U.S. Patent Application No. 2003/064133.

In the preferred practice of the invention, the DHA and rosemary containing beadlets described herein will be prepared by the following process:
(a) One part DHA oil containing 40% DHA is mixed with 0.2 parts of rosemary extract and the solution is heated to 40° C;
(b) At least one surfactant, such as ethoxylated mono and diglycerides, is added to the DHA and rosemary mixture so that the total amount of surfactant is about 0.2 parts kept at 40°C and produces a clear single-phase mixture of these oily components.
(c) The mixture of DHA, rosemary and surfactant is added to an aqueous solution prepared by dissolving one part sodium alginate and 0.5 parts protein with 0.08 parts of a second surfactant, such as sugar ester, in 70 parts of water at a temperature of 40° C for complete dissolution The mixture is mixed vigorously to produce an emulsion of alginate-protein-surfactants-DHA-rosemary. The micro droplets of the DHA oil and rosemary extract are encapsulated in the emulsion with the surfactants, protein and alginate in the water.
(d) This alginate-protein-surfactants-DHA-rosemary emulsion is added dropwise to a solution containing Ca²⁺, to form beadlets with a multilayered calcium alginate coating over the DHA- rosemary oil droplets which themselves have a coating of surfactants and protein;
(e) The calcium alginate-protein-surfactants-DHA-rosemary beadlets are removed by filtration from the Ca²⁺-containing solution, rinsed with an acidic solution and dried; and
(f) The dried calcium alginate-protein-surfactant-DHA-rosemary beadlets are coated with a rosemary-containing polymeric coating to obtain the final beadlets. The coating of the polymer solution containing the rosemary is sprayed on the calcium alginate-protein-surfactants-DHA-rosemary beadlets, in a fluidized bed coater.

The process described above provides a microencapsulated composition comprising DHA and rosemary enveloped by a surfactant and protein which is encapsulated in an alginate matrix and further coated with a film of cellulose derivative, which may contain rosemary extract. In certain embodiments, other kinds of outer coatings known in the art may be used.

The beadlets produced by this process are stable and possess a relatively large loading capacity of the active compounds. Stability of the beadlet in the present context refers to good containment, i.e. the encapsulation protects the active compound(s) from exposure to oxidation and other conditions which may adversely effect the compound. The rosemary both within the same beadlet as the DHA and in the surface coating provides even further protection of the DHA from oxidation and other conditions which may cause the production of the characteristic unpleasant "marine" odor and provides a sufficient amount of rosemary to provide ocular protection.

While one of skill in the art might typically expect that the coatings on the beadlets of the present invention would adversely effect the bioavailability of the DHA and rosemary, the opposite is true. The beadlets are known to dissolve, and so will release their contents in the stomach and intestine, and those contents can now be expected to have a greater fraction of less oxidized, more useful forms of, DHA and rosemary. Encapsulating the DHA and rosemary combination, where the DHA oil and rosemary extract composition is emulsified to very fine droplets , and rinsing the alginate beadlets with an aqueous acidic solution before the final drying of the beadlets, provides a microcapsule wherein the DHA and rosemary display improved stability and increased availability.

According to the process for preparing a particular embodiment of the present invention, a solution containing 0.2-1.0% of at least one surfactant agent, 0.5-5% of DHA oil, 0.1-1.0% of rosemary extract and/or its components, and 0.5% - 3.0% of protein and fillers is prepared. This solution is typically in the form of an emulsion or microemulsion. The processing of the DHA and rosemary mixture with a surface-active agent (surfactant) creates a coating of the surfactant around the particles of the DHA and rosemary, *i*.*e*. a primary protective layer.

Next, a separate alkali metal alginate solution is prepared by dissolving an alkali metal alginate in water to provide a solution containing 0.5% to 10% of an alkali metal alginate, preferably 1.5% sodium alginate in water. The alkali metal alginate solution is mixed with the DHA and rosemary emulsion. The resulting solution is homogenizes to provide a substantially homogeneous emulsion or dispersion which is added drop-wise to a solution containing 0.2% to 5% of Ca²⁺, preferably 1.5% calcium chloride (CaCl₂). The drop-wise addition is carried out such that the drops are not bigger than 1000 µm. The term "drop-wise" is meant to encompass such processes as, for example, spraying the emulsion into a solution containing Ca²⁺, dropping the emulsion via syringes or columns into the solution containing Ca²⁺, and other known processes which produces "droplets." Upon contact of the drops with the Ca²⁺ solution, beadlets of DHA and rosemary-containing alginate are formed. This creates a second protective layer for the active ingredients.

The size of the beadlets can be controlled by controlling the size of the droplets. Preferably the size of the drops is adjusted so as to provide beadlets in the size range of about 50 µm to about 700 µm, preferably about 100 µm to about 400 µm. The beadlets are then separated from the solution by conventional separating means, e.g., screening, and rinsed with an aqueous acidic solution. The acidic solution is preferably a 0.1% to 10% solution of an acid selected from among a group consisting of citric, aspartic, acetic, ascorbic, lactic, phosphoric or hydrochloric acid. More preferably, the acidic solution is a 2.5% solution of citric acid or phosphoric acid in water. The rinsing with an acidic solution effects shrinkage of the beadlets and improves the bioavailability of the microencapsulated DHA and rosemary.

The beadlets are then dried according to drying methods known in the art, preferably, by fluidized bed drying. "Drying" refers to a process by which the water content is lowered to below about 10%. While a water content somewhat above 10% would provide adequate and operable beadlets within the scope of the present invention, a water content below about 10%, and preferably below about 5%, is preferred to avoid the presence of microbial contamination within the beadlets and to provide a firmer beadlet. Of course, those of skill in the art will understand that the phrase "below about 10%" encompasses water content below about 9%, below about 8%, below about 7%, below about 6%, etc., including integral amounts there between.

The dry beadlets are then coated with a coating material in a fluidized bed apparatus, according to the coating technique described in U.S. Patent No. 4,710,384. Hence, a third coating layer is provided. This third layer provides further protection to the active ingredients inside the beadlet. Suitable coating materials for the final coating stage are cellulose derivatives, waxes, fats, proteins or polysaccharides, all optionally mixed with rosemary extract dissolved in a solvent. Non-limiting examples of cellulose derivatives suitable for coating material are: ethyl cellulose, hydroxy propyl cellulose, hydroxy propyl methylcellulose and methylcellulose. Waxes can be carnauba wax, candelila wax and beeswax. Fats can be palmitic and stearic acids as well as hydrogenated vegetables oils or different glycerides. Proteins can be albumins, zein, soy proteins or milk proteins. Polysaccharides can be starches, maltodextrins, pectins, xanthan gum, gum Arabic or carrageenan. According to a particular embodiment of the present invention, wherein there is no restriction regarding the use of products derived from animals, gelatin may be applied as a suitable protein for the third layer coating. In other preferred embodiment, soy proteins are used in the third layer coating. The final coating will further typically contain rosemary. It is preferred that the texture of the final coating is not sticky or tacky. Therefore, the type and form of rosemary used in the final coating will be chosen so as to avoid a sticky or tacky outer coating. One preferred form of rosemary for use in the outer coating is Lyc-o-rose.

The operation of size reduction in the first step of the process for preparing the beadlets of the present invention may be effected in a liquid medium. The liquid medium may be water or other water miscible solvents wherein non-limiting examples of suitable liquids include alcohols, e.g., methanol, ethanol, isopropanol, acetone and ethyl acetate. Accordingly, the liquid is added to the solution of the first step, which contains the DHA and rosemary mixture and surface active agent. When the DHA and rosemary mixture is in liquid form, it is preferred that the drop size of the DHA and rosemary mixture is reduced to a size not greater than 20 µm, preferably in the range of 0.1 µm to 7 µm, and the solution obtained is an emulsion.

It is contemplated that the drying and final coating may alternately be carried out in one step in a fluidized bed apparatus. Suitable fillers for use in the beadlets of the invention include, but are not limited to, polysaccharides, e.g., pectin, starch, carrageenan, gum Arabic, xanthan gum, carboxymethyl cellulose, methylcellulose, and hydroxypropyl cellulose.

The term "surfactant agent" refers to any substance that has emulsifying, colloidal, stabilizing or dispersing qualities. Non-limiting examples of suitable surfactants can be non-ionic, anionic or cationic surfactants, e.g., alcohol alkoxylates; alcohol ethoxylates; alkylphenol alkoxylates; alkylphenol ethoxylates; alkyl polysaccharides; block copolymers e.g., ethoxylated polypropylene oxides, alcoxylated ethylene diamine; esters e.g., glycerol mono and distearate, glycerol mono and dioleate; ethyoxylated sorbitan esters and sorbitan esters; and different proteins e.g., protein from soybean, corn or whey.

According to a particular embodiment of the present invention, the microencapsulated DHA and rosemary-containing composition contains 0.1% to 40% of DHA emulsion, wherein the particle size of the DHA emulsion is no greater than 20 µm, preferably between 0.1 µm and 7 µm. The composition may further include a surfactant, an alginate matrix which incorporates the surfactant-coated DHA and rosemary mixture and a coating which coats the DHA and rosemary-containing alginate matrix. The coating material is selected from among a group consisting of cellulose derivatives, waxes, fats, proteins and polysaccharides, all optionally combined with rosemary extract.

The microencapsulated compositions of the present invention are suitable for tablet preparation, hard shell capsule filling and incorporating in different foods. According to a particular embodiment of the present invention, the DHA and rosemary mixtures employed herein may further comprise proteins, fillers, excipients or additives. Examples of suitable fillers include starch, pectins, carrageenans, xanthan gums, proteins, polyethylene glycols, cellulose derivatives (e.g., methylcellulose, hydroxypropyl cellulose and ethyl cellulose) and other polysaccharides.

The preferred microcapsules or beadlets of the invention include three protective layers which protect the DHA and rosemary mixture. The first protective layer is created by the surfactant; the second protective layer is created by the alginate matrix and the third layer is the final coating layer.

The present invention is advantageous in that it provides microcapsules, or beadlets, containing DHA and rosemary, having no offensive odor, improved stability, relatively high content of the active agents and increased availability of the active agents. These advantages are achieved in one aspect by the process which provides a three layer coating of the DHA and rosemary and by the small particle size of said components. However, it is also contemplated that other beadlets containing the DHA and rosemary mixtures of the invention prepared by other means well-known in the art are within the scope of the present invention. The advantages of the present invention derive from the presence of the DHA and rosemary mixtures together in the same beadlet, thus decreasing or eliminating offensive odors and increasing stability and shelf life. The microcapsules of the present invention are tablet grade, *i*.*e*., suitable for use in tableting. The present invention further provides tablets comprising the beadlets described herein. Such tablets may be prepared by methods well known in the art. Preferred compositions of the present invention are gelatin free and are suitable for vegetarians.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result.

### Example 1

An example of the composition of the DHA/rosemary co-beadlet of the invention:

| | |
|---|---|
| DHA oil (40% DHA): | 24 % |
| Sodium alginate: | 24.8% |
| Isolated soy protein: | 16.5% |
| Silicon dioxide: | 2 % |
| Hydroxypropyl cellulose (Klucel) | 14.5% |
| Herbalox type O (rosemary) | 4.8% |
| Lyc-o-rose (rosemary): | 1.9% |
| Ethoxylated glycerides: | 4.8% |
| Sucrose ester: | 1.9% |
| Water and Ca: | ∼5% |
| Ca: Water | ∼1:5 |

### Example 2

An example of the composition of a second DHA/rosemary co-beadlet of the invention:

| | |
|---|---|
| DHA oil (40% DHA): | 20% |
| Sodium alginate: | 30% |
| Isolated soy protein: | 15% |
| Hydroxypropyl cellulose (Klucel) | 10% |
| | |
| Rosemary: | 6-8% |
| Ethoxylated glycerides: | 6% |
| Sucrose ester: | 6% |
| Water and Ca: | ∼5% |
| Ca : Water | ∼ 1: 5 |

### Example 3

An example of a tablet formulation containing a DHA/rosemary co-beadlet of the invention:

| **Micronutrient** | **Unit** | **Amount** |
|---|---|---|
| Vitamin A (β-carotene) | IU / mg | 2,500 /1.5 |
| Vitamin B-2 | mg | 5 |
| Folate | µg | 100 |
| Vitamin B-12 | mg | 3 |
| Vitamin C | mg | 200 |
| Vitamin E | IU | 75 |
| Copper | Mg | 0.5 |
| Manganese | mg | 5 |
| Selenium | µg | 20 |
| Zinc (acetate) | mg | 7.5 (eq. 12 oxide) |
| Lutein | mg | 2 |
| Zeaxanthin | mg | 1 |
| DHA (docosa- | mg | 5 |
| Hexaenoic acid [22:6ω3]) | | |
| Rosemary | mg | 7 |
| Excipients | mg | q.d. ∼ 950 |

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept, spirit and scope of the invention. More specifically, it will be apparent that certain agents which are both chemically and structurally related may be substituted for the agents described herein to achieve similar results.

### References

The following references, to the extent that they provide exemplary procedural or other details supplementary to those set forth herein, are specifically incorporated herein by reference.

### United States Patents

3,998,753
4,254,100
4,670,247

### Other References

E. E. Birch, S. Garfield, D. R. Hoffman, R. Uauy and D. G. Birch, "A randomized controlled trial of early dietary supply of long-chain polyunsaturated fatty acids and mental development in term infants", Developmental Medicine & Child Neurology 42:174-181 (2000).

## Claims

1. A beadlet comprising DHA and rosemary, said beadlet prepared by the following process:
(a) obtaining a mixture of DHA oil and rosemary extract;
(b) reducing the oil droplet size of the DHA and rosemary in said mixture in the presence of a surface active agent, thus creating an emulsion;
(c) obtaining a solution of alkali metal alginate and protein;
(d) combining the solution of step (b) with the solution of step (c);
(e) adding dropwise the solution obtained in step (d) to a solution containing Ca²⁺ to obtain beadlets;
(f) removing said beadlets from the solution;
(g) rinsing the beadlets with an acidic solution;
(h) drying the beadlets; and
(i) coating the beadlets.

2. A tablet comprising the beadlet of claim 1.

3. The tablet of claim 2, further comprising fillers, binders, disintegrants or other excipients for improving product or manufacturing characteristics.

4. The tablet of claim 2, wherein the amount of DHA in the tablet is 0.1% to 50% and the amount of rosemary in the tablet is 0.1% to 50%.

## Patentansprüche

1. Perlchen, umfassend DHA (Docosahexaensäure) und Rosmarin, welches Perlchen hergestellt ist durch das folgende Verfahren:
(a) Erhalt einer Mischung aus DHA-Öl und Rosmarin-Extrakt;
(b) Reduktion der Öltröpfchengröße des DHA und des Rosmarins in der Mischung in Anwesenheit eines oberflächenaktiven Agens, wobei eine Emulsion geschaffen wird;
(c) Erhalt einer Lösung von Alkalimetallalginat und Protein;
(d) Vereinigen der Lösung aus Schritt (b) mit der Lösung aus Schritt (c);
(e) tropfenweise Zugabe der in Schritt (d) erhaltenen Lösung zu einer Lösung, die Ca²⁺-enthält, zum Erhalt von Perlchen;
(f) Entfernen der Perlchen aus der Lösung;
(g) Spülen der Perlchen mit einer sauren Lösung;
(h) Trocknen der Perlchen; und
(i) Beschichten der Perlchen.

2. Tablette umfassend das Perlchen nach Anspruch 1.

3. Tablette nach Anspruch 2, welche weiters Füllstoffe, Bindemittel, Zerfallsstoffe oder andere Exzipienten umfasst zur Verbesserung des Produkts oder der Herstellungsmerkmale.

4. Tablette nach Anspruch 2, wobei die Menge an DHA in der Tablette 0,1% bis 50% beträgt und die Menge an Rosmarin in der Tablette 0,1% bis 50% beträgt.

## Revendications

1. Granule comprenant du DHA et du romarin, ledit granule étant préparé par le procédé suivant consistant à :
(a) obtenir un mélange d'huile de DHA et d'extrait de romarin ;
(b) réduire la taille des gouttelettes d'huile du DHA et du romarin dans ledit mélange en présence d'un agent tensioactif, créant ainsi une émulsion ;
(c) obtenir une solution d'alginate de métal alcalin et de protéine ;
(d) combiner la solution de l'étape (b) avec la solution de l'étape (c) ;
(e) ajouter goutte à goutte la solution obtenue dans l'étape (d) à une solution contenant des ions Ca²⁺ pour obtenir des granules ;
(f) retirer lesdits granules de la solution ;
(g) rincer les granules avec une solution acide ;
(h) sécher les granules ; et
(i) enrober les granules.

2. Comprimé comprenant le granule selon la revendication 1.

3. Comprimé selon la revendication 2, comprenant en outre des charges, des liants, des délitants ou d'autres excipients pour améliorer les caractéristiques de produit ou de fabrication.

4. Comprimé selon la revendication 2, dans lequel la quantité de DHA dans le comprimé est de 0,1 % à 50 % et la quantité de romarin dans le comprimé est de 0,1%à50%.
